# EUROPÄISCHE PATENTANMELDUNG

(11) **EP 4 470 392 A1**
(43) Veröffentlichungstag der Anmeldung: **04.12.2024**
(21) Anmeldenummer: 23176955.5
(22) Anmeldetag: 02.06.2023
(51) Int. Cl.: A23L 33/10, A23L 33/13, A61K 31/455, A61K 31/7084, A61P 25/00, A23L 33/15

(54) **NICHT-THERAPEUTISCHE VERWENDUNG EINES NADH-PRÄPARATS IN KOMBINATION MIT SPORT**

(71) Anmelder: Nutropia Group GmbH, 5585 Unternberg (AT)
(72) Erfinder: FUCHS, Norbert, 5580 Unternberg (AT); RÖSLER, Dietmar, 5571 Mariapfarr (AT)
(74) Vertreter: SONN Patentanwälte GmbH & Co KG

(57) **Zusammenfassung**

Offenbart wird die Verwendung eines NADH-Präparats in Kombination mit einer Sportintervention zur Erhöhung der kognitiven Leistungsfähigkeit oder zur Verbesserung des allgemeinen Gesundheitszustands. Die durch Einnahme des NADH-Präparats zugeführte NADH-Tagesdosis beträgt bevorzugt 1 mg - 100 mg. Die Sportintervention kann Herz-Kreislauftraining und Krafttraining umfassen.

## Beschreibung

Das Gebiet der vorliegenden Erfindung ist das der Verwendungen von Nahrungsergänzungsmitteln, insbesondere NADH-Präparaten.

Das Reduktionsäquivalent NADH (Nikotinamid-Adenin-Dinukleotid-Hydrid) ist an zahlreichen Stoffwechselreaktionen beteiligt und dient als Elektronen- und Protonendonator. Als Coenzym und temporärer Interaktionspartner für Oxidoreduktasen geht das wasserlösliche NADH reversible Bindungen mit diesen Enzymen ein und spielt dabei eine Schlüsselrolle bei der Regulierung zahlreicher biologischer Prozesse. In seiner Funktion als Elektronentransporter nimmt es Elektronen aus Stoffwechselprozessen wie beispielsweise Citratzyklus, beta-Oxidation und Glykolyse auf und überträgt diese in den Mitochondrien im Rahmen der oxidativen Phosphorylierung über die NADH-Dehydrogenase auf Ubichinon-10 und letztendlich auf den Atemsauerstoff. Daher ist NADH maßgeblich am Energiestoffwechsel durch die Beteiligung an der Bildung von Adenosin-5'-Triphosphat (ATP) als "biochemischer Universalwährung" involviert.

NADH-Präparate werden als Nahrungsergänzungsmittel eingesetzt, weil sie dazu beitragen, den natürlichen NADH-Spiegel im Körper zu erhöhen. Die Rolle von NADH im menschlichen Stoffwechsel ist grundsätzlich zwar einigermaßen erforscht, aber die Auswirkungen von NADH-Supplementen auf den menschlichen Körper sind weiterhin Gegenstand laufender Untersuchungen. NADH-Präparate werden oft zur Verbesserung der kognitiven Funktion eingesetzt. NADH-Präparate können auch dazu beitragen, den altersbedingten Rückgang der Energieproduktion in den Körperzellen zu vermindern.

NADH-Präparate haben sich auf dem deutschen Markt als Nahrungsergänzungsmittel etabliert und werden in verschiedenen Formen angeboten, darunter Tabletten und Kapseln. Diese Präparate sind in verschiedenen Dosierungen erhältlich, wobei einige Produkte beispielsweise 1, 2, 5, 10 oder 20 Milligramm NADH pro Tablette oder Kapsel enthalten. Die Wahl der Dosierung kann abhängig von individuellen Bedürfnissen und Empfehlungen von Gesundheitsdienstleistern variieren.

So wird beispielsweise das NADH-Präparat PANMOL^{®} NADH unter anderem zur Verbesserung der Gedächtnis- und Konzentrationsleistung und zur Erhöhung der Wachsamkeit bzw. Aufmerksamkeit eingesetzt.

Es besteht jedoch allgemein der Bedarf, die Wirksamkeit von NADH-Präparaten weiter zu erhöhen. Daher ist es Aufgabe der vorliegenden Erfindung, eine neue Verwendung zur Verfügung zu stellen, aufgrund derer positive Wirkungen von NADH-Präparaten verstärkt werden.

Die vorliegende Erfindung stellt die (nicht-medizinische) Verwendung eines NADH-Präparats (bei gesunden Personen) in Kombination mit einer Sportintervention zur Erhöhung der kognitiven Leistungsfähigkeit (insbesondere der motorisch-kognitiven Leistungsfähigkeit) oder zur Verbesserung des allgemeinen Gesundheitszustands zur Verfügung.

Im Zuge der vorliegenden Erfindung stellte sich heraus, dass die Kombination der NADH-Supplementation mit einer Sportintervention auf synergistische Weise zu hervorragenden Ergebnissen sowohl bei der Verbesserung der kognitiven Leistungsfähigkeit (insbesondere die Reaktionsfähigkeit, wie sie beispielsweise vom sogenannten Shape Jump-Test ermittelt wird, vgl. das Beispiel unten) als auch bei der Verbesserung des allgemeinen Gesundheitszustands (beispielsweise ermittelt durch den etablierten SF-36 Gesundheitsfragebogen) führt.

Herz-Kreislauf-Training, auch bekannt als Ausdauer- oder Cardiotraining, ist eine Art von körperlicher Aktivität, die das Herz-Kreislauf-System stärkt und die allgemeine körperliche Ausdauer verbessert. Es umfasst Aktivitäten, die über einen längeren Zeitraum ausgeführt werden und bei denen mehrere große Muskelgruppen beteiligt sind, wie Laufen, Radfahren, Schwimmen oder Wandern.

Krafttraining, auch bekannt als Widerstands- oder Gewichtstraining, ist hingegen eine Art von körperlicher Aktivität, die darauf abzielt, die Stärke und Größe der Muskulatur durch verschiedene Übungen zu verbessern, die bestimmte Muskelgruppen gegen einen Widerstand arbeiten lassen. Dies kann durch das Heben von Gewichten, das Arbeiten mit Widerstandsbändern oder das Durchführen von Körpergewichtsübungen erreicht werden.

In einer bevorzugten Ausführungsform umfasst die Sportintervention Herz-Kreislauftraining und/oder Krafttraining. Es hat sich herausgestellt, dass diese beiden Trainingsformen besonders gut synergetisch mit dem NADH-Präparat zusammenwirken, um die erfindungsgemäßen Wirkungen zu erreichen.

Es ist besonders vorteilhaft, wenn die Sportintervention mehrere Trainingseinheiten aufweist. Bevorzugt sind zumindest 4 Einheiten innerhalb von 4 Wochen (oder auch einfach: zumindest eine Einheit innerhalb von 4 Wochen), mehr bevorzugt zumindest 6 Einheiten innerhalb von 4 Wochen, noch mehr bevorzugt zumindest 8 Einheiten innerhalb von 4 Wochen. Eine Trainingseinheit weist bevorzugt zumindest 30 Minuten, bevorzugt zumindest 50 Minuten, Sporttraining auf, beispielsweise sowohl Herz-Kreislauftraining als Krafttraining (z.B. 20 Minuten Herz-Kreislauf-Training und 35 Minuten Krafttraining).

Gemäß einer bevorzugten Ausführungsform wird das NADH-Präparat an einer erwachsenen Person angewendet. Erwachsene profitieren besonders von der Verwendung des NADH-Präparats in Verbindung mit einer Sportintervention.

Vorzugsweise ist die Person, an der das NADH-Präparat angewendet wird, gesund. Das bedeutet im erfindungsgemäßen Zusammenhang, dass zumindest keine Stoffwechselstörung (wie z.B. Diabetes mellitus) und keine neurologische Grunderkrankung (wie z.B. Morbus Parkinson) vorliegt. Insbesondere bedeutet es, dass überhaupt keine Krankheit oder Störung an der Person vorliegt.

Wie sich herausgestellt hat, profitieren untrainierte (gesunde) Personen ganz besonders von der erfindungsgemäßen Verwendung. Als "untrainiert" wird eine Person verstanden, wenn sie wöchentlich weniger als 90 Minuten Sport betreibt, bevorzugt weniger als 60 Minuten, insbesondere weniger als 30 Minuten. Daher ist in einer bevorzugten Ausführungsform die Person, an der das NADH-Präparat angewendet wird, untrainiert (und gesund).

Die Altersgruppe ab 30 profitiert besonders von der Kombination aus NADH-Präparat und Sportintervention. Mit zunehmendem Alter können Veränderungen im Energiehaushalt und in der kognitiven Leistungsfähigkeit auftreten. Daher ist die Person, an der das NADH-Präparat angewendet wird, vorzugsweise über 30 Jahre, bevorzugt über 40 Jahre, insbesondere über 50 Jahre alt.

In einer bevorzugten Ausführungsform beträgt die durch Einnahme des NADH-Präparats zugeführte NADH-Tagesdosis zwischen 1 mg und 100 mg - in diesem Bereich werden besonders gute Ergebnisse erzielt (vor allem trifft dies auf den Bereich von 1 mg - 20 mg NADH-Tagesdosis zu). Vorzugsweise beträgt die zugeführte Tagesdosis 2 mg - 75 mg, mehr bevorzugt 5 mg - 50 mg, noch mehr bevorzugt 6 mg - 25 mg, insbesondere 7 mg - 15 mg oder gar 8 mg - 12 mg. In einer weiteren bevorzugten Ausführungsform beträgt die zugeführte Tagesdosis 1 mg - 20 mg, mehr bevorzugt 2 mg - 18 mg, noch mehr bevorzugt 4 mg - 16 mg, noch mehr bevorzugt 6 mg - 14 mg, insbesondere 8 mg - 12 mg.

In einer weiteren bevorzugten Ausführungsform werden der Person, die das NADH-Präparat einnimmt, keine weiteren Nahrungsergänzungsmittel-Präparate zugeführt. Dies kann besonders vorteilhaft sein, um unerwünschte Interaktionen mit anderen Nahrungsergänzungsmitteln möglichst zu vermeiden und so die Wirkungen des NADH-Präparats zu maximieren.

Das NADH-Präparat wird vorzugsweise oral verabreicht.

Gemäß einer weiteren bevorzugten Ausführungsform umfasst das NADH-Präparat zumindest ein weiteres Antioxidans (zur Stabilisierung des NADH-Präparats). Geeignete Antioxidantien sind beispielsweise Vitamin C (Ascorbinsäure), Vitamin E (Tocopherol), Beta-Carotin oder Flavonoide wie Resveratrol oder Quercetin. Ganz besonders gut geeignet sind Chlorophyll oder (reduziertes) Ferredoxin.

Chlorophylle weisen derivatisierte Porphyrin-Ringe mit Magnesium (Mg²⁺) aus chelatartig gebundenem Mineralstoff auf. Chlorophylle besitzen strukturelle Ähnlichkeiten mit den Eisen-haltigen Hämen, die in tierischen und humanen Organismen elementare Stoffwechselfunktionen als Hämoglobin, Myoglobin und Cytochrome erfüllen. Während Häme überwiegend dem Sauerstoff- und Elektronentransport dienen, sind Chlorophylle als einzige Vertreter dieser Porphyrine in der Lage, Lichtquanten aus der Sonnenstrahlung zu absorbieren und so über die Energie des Lichtes den pflanzlichen Organismus dazu anzuregen, aus CO₂ und H₂O organische Moleküle und Sauerstoff zu synthetisieren (Photosynthese). Während die Energiegewinnung tierischer und humaner Zellen durch den Abbau dieser pflanzlichen, organischen Moleküle und über den schrittweisen Auf- und Abbau gespeicherter Elektronen-Energie (über die sogenannten Reduktionsäquivalente NADH/H+ und FADH2) analog zum biochemischen Energie-Gefälle der Kalorienträger abläuft, kommt dem Chlorophyll die Aufgabe zu, die Energie des Sonnenlichtes in elektronische Energie zu transformieren und so dem Aufbau neuer organischer Substanz zur Verfügung zu stellen. Eine physiologisch dem NADH ähnliche, wenn auch elektrochemisch diametrale, Rolle als Elektronen-Spender erfüllt Chlorophyll im pflanzlichen, vielleicht auch humanen Organismus. So verweisen zahlreiche pflanzenphysiologische Publikationen auf die Zusammenhänge von Chlorophyll-Gehalten und -Aktivitäten auf die Dynamik des Pflanzenwachstums. Auch weisen neuere Publikationen auf Zusammenhänge einer Chlorophyll-Versorgung und dem Stoffwechsel der Katecholamine hin.

Ferredoxine hingegen sind eisenhaltige Proteine, die eine entscheidende Rolle in den biochemischen Redoxprozessen in Zellen spielen. Sie dienen als Zwischenüberträger von Elektronen in einer Vielzahl von metabolischen Reaktionen, einschließlich der Photosynthese, der Atmung und der Stickstofffixierung. In der Photosynthese zum Beispiel übertragen Ferredoxine Elektronen, die durch Lichtenergie erzeugt wurden, auf andere Moleküle im Prozess der Kohlenstoffdioxidreduktion. Ferredoxine sind in allen Organismen zu finden, von Bakterien und Algen bis hin zu Pflanzen und Tieren.

Gemäß einer weiteren Ausführungsform beinhaltet das NADH-Präparat Bienenwachs. Bienenwachs ist ein natürliches Wachs, das von Honigbienen produziert wird. Bienenwachs dient als natürlicher Stabilisator und kann dadurch die Haltbarkeit des Präparats erhöhen. Darüber hinaus kann Bienenwachs unter anderem die Freisetzung von NADH im Körper der Person verbessern und führt zu einer Erhöhung der Wirksamkeit.

In einer weiteren bevorzugten Ausführungsform weist das NADH-Präparat pflanzliches Fett auf. Dieses verbessert unter anderem die Bioverfügbarkeit des NADH im Körper der Person und führt zu einer Erhöhung der Wirksamkeit.

Gemäß einer weiteren bevorzugten Ausführungsform liegt das NADH-Präparat in fester Form vor, insbesondere als Kapsel oder Tablette. Diese feste Darreichungsform ermöglicht eine einfache und genaue Dosierung des NADH-Präparats, was zu einer konsistenten Aufnahme der Substanz über die Dauer der Anwendung und damit über die Zeit zu einer noch zuverlässigeren Wirkung führt.

In einer weiteren bevorzugten Ausführungsform weist das NADH-Präparat eine mikroverkapselte Formulierung auf. Diese erhöht die Stabilität weiter, indem NADH vor Umwelteinflüssen wie Feuchtigkeit, Hitze oder Licht geschützt wird. Zudem kann die mikroverkapselte Formulierung eine kontrollierte Freisetzung des NADH ermöglichen. Bei der Mikroverkapselung wird die aktive Komponente - im vorliegenden Fall zumindest das NADH - in Mikrokapseln eingebettet, deren Durchmesser üblicherweise im Bereich von 1 µm bis 500 µm liegt (beispielsweise durch Elektronenmikroskopie bestimmt).

In einer weiteren bevorzugten Ausführungsform ist das NADH-Präparat magensaftresistent formuliert. Dadurch wird gewährleistet, dass das NADH das aggressive Milieu des Magens unbeschädigt übersteht und erst im Dünndarm freigesetzt wird, was die Wirksamkeit weiter erhöht.

In einer weiteren bevorzugten Ausführungsform dauert die Verwendung des NADH-Präparats über zumindest 5 Tage, mehr bevorzugt über zumindest 10 Tage, noch mehr bevorzugt über zumindest 20 Tage und insbesondere über zumindest 30 Tage an. Dadurch werden nachhaltigere gesundheitliche bzw. kognitive Vorteile erzielt, wie sich herausgestellt hat.

Es gibt verschiedene anerkannte Methoden, um den allgemeinen Gesundheitszustand von Personen zu beurteilen. Eine der am häufigsten verwendeten Methoden ist der SF-36 Gesundheitsfragebogen, insbesondere das Fragen-Subset (Skala) der "Allgemeinen Gesundheitswahrnehmung". Der SF-36 Gesundheitsfragebogen ist ein generisches Instrument zur Messung der gesundheitsbezogenen Lebensqualität. Er besteht aus 36 Fragen, die in acht verschiedene Skalen unterteilt sind: Körperliche Funktionsfähigkeit, Körperliche Rollenfunktion, Körperliche Schmerzen, Allgemeine Gesundheitswahrnehmung, Vitalität, Soziale Funktionsfähigkeit, Emotionale Rollenfunktion und Geistige Gesundheit. Die Antworten auf diese Fragen werden dann in einen Gesamtscore umgerechnet. Der SF-36 ist bekannt für seine hohe Zuverlässigkeit und Validität und wird häufig in klinischen Studien und Forschungsprojekten verwendet, um die Wirksamkeit von Interventionen zu bewerten (Ware Jr, John E., and Cathy Donald Sherbourne. "The MOS 36-item short-form health survey (SF-36): I. Conceptual framework and item selection." Medical care (1992): 473-483.).

Darüber hinaus gibt es auch andere Methoden zur Beurteilung des Gesundheitszustands, wie z.B. Biomarker-Tests, wie z.B. den Blutzuckerspiegel, den Cholesterinspiegel oder die Anzahl der roten und weißen Blutkörperchen. Auch diese Messungen können herangezogen werden, um den allgemeinen Gesundheitszustand einer Person zu beurteilen. Hierin bevorzugt ist jedoch der SF-36 Gesundheitsfragebogen, insbesondere die Skala der "Allgemeinen Gesundheitswahrnehmung".

Die kognitive Leistungsfähigkeit (insbesondere die motorischkognitive Leistungsfähigkeit) wird hierin bevorzugt mit dem SKILLCOURT^{®}-System ermittelt (Friebe, David, et al. "Reliability and Usefulness of the Skillcourt as a Computerized Agility and Motor-Cognitive Testing Tool." Medicine and Science in Sports and Exercise (2023).; Hülsdünker, Thorben, et al. "Validity of the SKILLCOURT® technology for agility and cognitive performance assessment in healthy active adults." Journal of Exercise Science & Fitness (2023)). Hierbei hat sich insbesondere der "Shape Jump"-Test dieses Systems bewährt. Beim "Shape Jump"-Test wird die Reaktionsgeschwindigkeit, mit der korrekt auf neue Reize (auf einem Bildschirm angezeigte Formen - Shapes) durch eine Bewegung (Sprung) reagiert wird, beurteilt. Ein weiteres Verfahren zur Ermittlung der kognitiven Leistungsfähigkeit ist beispielsweise der D2-Test der Aufmerksamkeit (D2R), vgl. Bates & Lemay. "The d2 Test of attention: construct validity and extensions in scoring techniques." Journal of the International Neuropsychological Society 10.3 (2004): 392-400.

Im Folgenden wird die Erfindung anhand von bevorzugten, nicht einschränkenden Beispielen und Figuren näher erläutert.

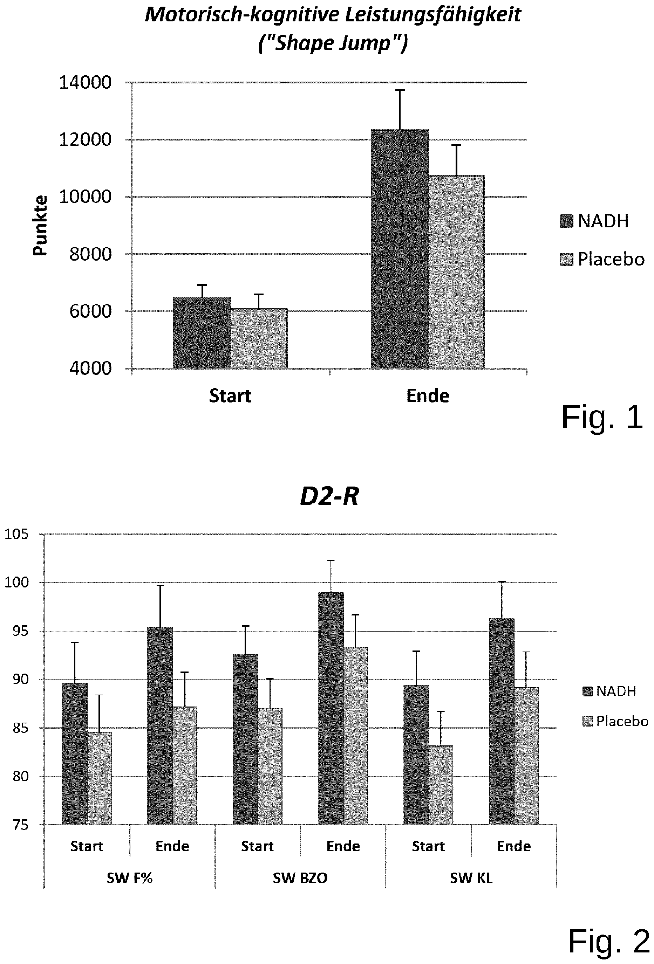

Fig. 1 zeigt die Ergebnisse des "Shape Jump"-Tests im SkillCourt^{®}-System, der die motorisch-kognitiven Fähigkeiten der Probanden misst, zu Beginn und, vier Wochen später, zum Endzeitpunkt der Studie für die Verum- (NADH-Supplementation und Sportintervention) wie auch die Placebogruppe (Sportintervention ohne NADH-Supplementation). Bei den Punkten handelt sich um willkürliche Einheiten.

Fig. 2 zeigt Standardwerte (SW) der Fehlerrate (F%), der Schnelligkeit (BZO) und der Konzentrationsleistung (KL) beider Prüfgruppen (NADH-Supplementation und Sportintervention, Sportintervention ohne NADH-Supplementation) nach Alterskorrektur. Der Prüfzeitraum umfasste vier Wochen.

### Beispiel - Randomisierte Doppelblindstudie

Ziel dieser prospektiven randomisierten Placebo-kontrollierten Doppelblindstudie war die Erhebung der Wirksamkeit einer vierwöchigen NADH-Supplementation mit Sportintervention auf kognitive Eigenschaften und Gesundheitszustand eines untrainierten Kollektivs. 27 untrainierte gesunde Erwachsene wurden herangezogen, um für die Dauer von vier Wochen an einem gezieltem Trainingsprogramm teilzunehmen und zugleich entweder je 10 mg/Tag Chlorophyll-NADH (n =13; Alter: 40,31 ± 10,49 Jahre) oder ein Chlorophyll-haltiges Vergleichs-Präparat (n = 14; Alter: 43,71 ± 12,96 Jahre) einzunehmen. Jeweils zu Beginn der Studie und nach vier Wochen wurden die Probanden/- innen einigen Testungen unterzogen: mit dem *D2-R*-Test wurde die Aufmerksamkeit und Konzentrationsfähigkeit der Probanden/-innen überprüft, die kognitiv-motorischen Fähigkeiten (*Shape Jump*) wurden mittels des *SkillCourt^{®},* eines digitalen Prüfsystems mit Scannertechnologie, gemessen und mit dem validen Testverfahren *SF-36* wurde der Gesundheitszustand ermittelt. Im *D2-R* Test schnitt die Chlorophyll-NADH-Gruppe besser ab als die Vergleichs-Gruppe. Vor allem die Fehlerhäufigkeit (Verringerung der Fehlerrate: 6,43%; Startwert: 89,62 ± 4,46; Endwert: 95,38 ± 4,61) war in dieser Gruppe verglichen zu der Kontrollgruppe verringert. In der Vergleichs-Gruppe betrug die Verbesserung lediglich 3,17% (Startwert: 84,5 ± 3,93; Endwert: 87,18 ± 3,60). Eine verbesserte Leistung im Testverfahren *"Shape Jump",* welches die Probanden/-innen der Verum-Gruppe entwickelten, bestätigt die Wirkung der erfindungsgemäßen Kombination die kognitive Leistung: In der Verum-Gruppe konnte die anfängliche Punktezahl von 6.475 ± 453,18 innerhalb von vier Wochen auf 12.347,73 ± 1.289,81 Punkte gesteigert werden, was einer Leistungssteigerung um 90,70% entsprach, wohingegen Teilnehmer/- innen der Vergleichs-Gruppe lediglich eine Verbesserung von 76,74% (Anfangspunktzahl: 6.072,92 ± 528,17; Endwert: 10.733,33 ± 1.075,43) erfuhren. Die Verbesserung des Gesamtgesundheitszustandes war mit einer Steigerung von 5,7% (Startpunktzahl: 125,3 ± 2,51; Endwert: 132,45 ± 2,05) in der Chlorophyll-NADH-Gruppe höher verglichen zu der Placebo-Gruppe (Verbesserung: 4,6%; Anfangswert: 126,35 ± 2,76; Endpunktzahl: 132,16 ± 2,1). Die erfindungsgemäße Kombination von NADH-Supplementation und Sportintervention resultierte in einer zusätzlichen Verbesserung des Gesamtgesundheitszustandes, sowie in einer Steigerung der kognitiven Fähigkeiten.

### Methoden

### Studiendesign und Probanden/-innen

In einer randomisierten, doppelblinden Pilotstudie wurde der Einfluss einer vierwöchigen Chlorophyll-NADH-Supplementierung im Vergleich zu einer alleinigen Chlorophyll-Supplementierung in Kombination mit einem vierwöchigen Trainingsprogramm auf die kognitive Leistung und den Gesundheitszustand an 28 gesunden Erwachsenen untersucht. Die Richtlinien der Deklaration von Helsinki wurden berücksichtigt. Die Studienteilnehmer/-innen wurden mündlich über das Projekt aufgeklärt und stimmten dem Projekt in schriftlicher Form mittels einer unterzeichneten Einverständniserklärung zu.

Gesunde untrainierte Männer und Frauen, die wöchentlich weniger als 3 x 30 Minuten regelmäßigen Sport betrieben, jedoch die Bereitschaft zur einem Trainingsprogramm mit 10 Einheiten von jeweils 60 Minuten Training innerhalb von 4 Wochen erklärten, erfüllten die Einschlusskriterien. Zu den Ausschlusskriterien gehörten eine niedrige Compliance (< 90%), die Einnahme von Medikamenten (mit Ausnahme von Kontrazeptiva) oder Vitamin-, Mineralstoff-, Fettsäure-Supplementation, die das Studienergebnis verfälschen könnten, in den letzten 4 Wochen, eine laufende Ernährungstherapie, eine vorliegende Stoffwechselstörung, bekannte Malabsorption, eine neurologische Grunderkrankung oder starke motorische Einschränkungen am Bewegungsapparat. Regelmäßiges Training (> 3 x 30 Minuten/Woche), Unverträglichkeiten gegen die Prüfsubstanz, eine vorliegende Schwangerschaft oder das Fehlen der Einwilligungserklärung führten ebenfalls zu einem Ausschluss aus der Studie. Das Ausmaß des Fitnessprogrammes umfasste zehn Einheiten innerhalb von vier Wochen. Eine Trainingseinheit dauerte 60 Minuten. Dabei wurde 20 Minuten ein moderates Herz-/Kreislauftraining, 35 Minuten ein Krafttraining und 5 Minuten ein kognitives Training durchgeführt. 32 Probanden/-innen wurden für die Studie rekrutiert. 28 Teilnehmer/-innen erfüllten die Einschlusskriterien und wurden per Zufallsprinzip mittels eines validierten Systems entweder der Verum-Gruppe, welche die Prüfsubstanz mit 10 mg NADH-Tagesdosis (in einer mikroverkapselten Formulierung) erhielten oder der Placebo-Gruppe, die ein entsprechendes Placebo-Präparat ausgehändigt bekamen, zugeordnet. Mit einem Verhältnis von 14:13 entsprach die Gruppengröße der Interventionsgruppe in etwa der Gruppengröße der Kontrollgruppe (Placebo). Die Teilnehmer/-innen wurden mittels Laufnummern anonymisiert. Ein Proband erkrankte während der Abschlussphase an COVID-19, so dass seine Daten nicht verwertbar waren.

Die beiden Prüfsubstanzen waren optisch nicht voneinander zu unterscheiden. Das Chlorophyll-NADH-Präparat (PANMOL^{®} NADH micro) enthielt Hilfsstoffe aus Erbsenfasern und mittelkettigen Kokos-Triglyceriden. Es war in einer Kapselhülle aus Rindergelatine der Größe 2 verkapselt, eine Kapsel entsprach einer Tagesdosis von 10 mg NADH und 10 mg Chlorophyll. Die Vergleichssubstanz enthielt indessen nur 10 mg Chlorophyll. Das Vergleichs-Präparat war optisch nicht vom Verum-Präparat zu unterscheiden. Jeweils 30 Kapseln der Prüfsubstanzen (entweder Chlorophyll-NADH oder Chlorophyll) waren in weißen Behältern verpackt. Die Behältnisse von Verum- und Placebo-Präparat waren optisch ident.

Eine Kapsel des stabilisierten, magensaftresistenten Chlorophyll-NADH-Mixes oder des entsprechenden Vergleichs-Produkts wurden morgens oral mit ausreichend Flüssigkeit konsumiert. Die erstmalige Einnahme erfolgt am Tag nach der Aufnahme in die Studie. Die Konsumation der Prüfsubstanz wurde die nächsten 30 Tage fortgeführt.

Zum Zeitpunkt T1, der Tag vor der ersten Einnahme der Prüfsubstanz, wurde der Anfangs-Check durchgeführt. Dieser umfasste die Erhebung des physischen und psychischen Gesundheitszustandes über den standarisierten *SF-36*-Fragebogen. Die Konzentration und Aufmerksamkeit wurden mittels des *D2-R-*Konzentrationstests ermittelt. Mit Hilfe des *SkillCourt*^{®}-Systems wurden die Parameter Reaktion, Konzentration und Motorik bestimmt.

Anschließend erhielten die Probanden/-innen die Prüfsubstanzen (Verum oder Placebo) ausgehändigt, über deren Wirkung und Einnahme sie im Vorfeld detailliert aufgeklärt wurden. Durch einen geschulten Fitnesstrainer wurden die Probanden/-innen ausführlich über die korrekte Benutzung der Fitnessgeräte unterrichtet und das Fitnessprogramm wurde erklärt. Ein Trainer betreute die Teilnehmer/-innen auch während der Sporteinheiten. Gesetztes Ziel waren zehn Trainingseinheiten während der nächsten vier Wochen. Nach dieser Periode (nach 28 Tagen) erfolgte ein Abschluss-Check, der erneut eine Abfrage des Gesundheitszustandes über den *SF-36,* die Durchführung des *D2-R-*Konzentrations- und Aufmerksamkeitstests und die Analyse von Motorik und Reaktionsfähigkeit (über den *SkillCourt*^{®}) inkludierte.

### D2-R

Der *D2-R*-Aufmerksamkeits- und Konzentrationstest ist ein validiertes Testverfahren zur Messung der individuellen Aufmerksamkeit und Konzentrationsfähigkeit (vgl. Bates, Marsha E., and Edward P. Lemay. "The d2 Test of attention: construct validity and extensions in scoring techniques." Journal of the International Neuropsychological Society 10.3 (2004): 392-400.). Der Testbogen besteht aus 14 Testzeilen mit jeweils 57 Zeichen. Die Teilnehmer/-innen wurden angewiesen, zwischen ähnlichen visuellen Reizen zu unterscheiden, indem sie die Zielobjekte ("d" mit zwei Strichen) identifizieren und durchstreichen sollten, wohingegen alle anderen Zeichen ("p" oder "d" mit weniger oder mehr als zwei Strichen) ignoriert werden sollten. Die Zeit für die Bearbeitung einer Zeile wurde dabei auf 20 Sekunden limitiert (Gesamtzeit für den Test: 4 min 40 s). Kennwerte des *D2-R*-Tests umfassen die Gesamtzahl der bearbeiteten Items, die Gesamtzahl der Fehler (Auslassungs- und Begehungsfehler), der prozentuale Anteil der Fehler im Verhältnis zu den bearbeiteten Items und ein Gesamtleistungswert der Aufmerksamkeits-/Konzentrationsfähigkeit. Die Reliabilität hängt von der getesteten Altersgruppe ab und liegt zwischen alpha=0,89 und 0,95. Durch Vergleiche mit einer Normtabelle, die Standardwerte ausgibt, erfolgt eine Alterskorrektur.

### SkillCourt^{®}

Der *Skillcourt*^{®} ist eine digitale Trainingsanlage mit Scannertechnologie, die sich zur Messung motorischer und kognitiver Parameter der Probanden/-innen eignet (vgl. Friebe, David, et al. "Reliability and Usefulness of the Skillcourt as a Computerized Agility and Motor-Cognitive Testing Tool." Medicine and Science in Sports and Exercise (2023).; Hülsdünker, Thorben, et al. "Validity of the SKILLCOURT® technology for agility and cognitive performance assessment in healthy active adults." Journal of Exercise Science & Fitness (2023)). Als Parameter wurden die kognitiven Eigenschaften und Reaktionsfähigkeit der Teilnehmer/-innen über den *Shape Jump-Test* überprüft: Über den Monitor wurde eine von drei möglichen Formen (Kugel, Pyramide oder Würfel) angezeigt. Diese befand sich zunächst in der Mitte von drei Bahnen. Auf jeder dieser Bahn war jeweils ein Hindernis zu finden mit einem Ausschnitt (für Kugel, Pyramide oder Würfel) durch den nur die entsprechende Form passte. Nun mussten die Teilnehmer/-innen durch Fußbewegungen diese Form so steuern, dass diese auf die richtige Bahn gebracht wurde, wo sich das Hindernis mit dem passenden Ausschnitt für die Form befand. Die unterschiedliche Einfärbung von Form zu passendem Tunnel erforderte eine zusätzliche Konzentrationsfähigkeit. Die Zeit für eine Reaktion war befristet. Pro Runde blieb den Teilnehmern/-innen weniger Zeit, die Aufgabe zu bearbeiten. Das System vergab automatisiert Punkte, je nachdem, wie fehlerfrei diese Übung von den Teilnehmern/-innen erfüllt werden konnte. Diese Punkte wurden notiert und für die statistische Auswertung verwendet.

### Verträglichkeit und Akzeptanz der Prüfsubstanzen

Als zusätzliche Sekundärparameter wurden Verträglichkeit und Akzeptanz der Prüfsubstanzen ermittelt. Dies geschah über ein ausgehändigtes Teilnehmertagebuch. Die Teilnehmer/-innen wurden aufgefordert, darin alle subjektiven Auffälligkeiten und Besonderheiten zu vermerken, die sie in einen möglichen Zusammenhang zu den Prüfsubstanzen brachten.

### SF-36

Der *SF-36* Health Survey ist ein krankheitsübergreifendes validiertes Messinstrument zur Erfassung der gesundheitsbezogenen Lebensqualität von Patienten/-innen, bestehend aus 36 Items (Ware Jr, John E., and Cathy Donald Sherbourne. "The MOS 36-item short-form health survey (SF-36): I. Conceptual framework and item selection." Medical care (1992): 473-483). Er umfasst acht Bereiche der subjektiven Gesundheit: Körperliche Funktionsfähigkeit, körperliche Rollenfunktion, körperliche Schmerzen, allgemeine Gesundheitswahrnehmung, Vitalität, soziale Funktionsfähigkeit, emotionale Rollenfunktion und psychisches Wohlbefinden, die sich den Grunddimensionen "körperliche und psychische Gesundheit" zuordnen lassen. Pro Item werden entsprechend dem individuellen Gesundheitszustandes Punkte vergeben, so dass sich der Gesundheitszustand mittels des *SF-36* quantifizieren lässt. Höhere Punktzahlen deuten auf eine bessere Lebensqualität hin. Maximal können 155 Punkte erreicht werden.

### Resultate

Für die gegenwärtigen Studie wurden 32 Teilnehmer/-innen rekrutiert. Die Aufteilung in die beiden Prüfgruppen (Verum oder Placebo) erfolgte mittels eines validierten Randomisierungssystems. Bei vier Personen kam es zu einem vorzeitigen Studienabbruch, so dass die endgültige Studienpopulation aus 28 gesunden Frauen (n = 19) und Männern (n = 9) im Alter zwischen 18 und 65 Jahren bestand. Aus der Endauswertung musste ein weiterer Teilnehmer aufgrund einer COVID-19 Erkrankung zum Zeitpunkt des Abschlusstests ausgeschlossen werden. Die Daten von 27 Teilnehmer/-innen gingen in die Auswertung ein. Die Gruppengröße beider Prüfgruppen war ähnlich groß: 13 Teilnehmer/-innen (neun Frauen, vier Männer) nahmen die Chlorophyll-NADH-Prüfsubstanz ein und 14 Probanden/- innen (neun Frauen, fünf Männer) erhielten das Vergleichs-Präparat. Das durchschnittliche Alter der NADH-Gruppe betrug 40,31 ± 10,49 Jahre und das Alter der Placebo-Gruppe ergab einen Mittelwert (± SD) von 43,71 ± 12,96 Jahre. Tabelle 1 zeigt die demografischen Merkmale der Teilnehmer/-innen. Die demografischen Parameter waren normalverteilt und es gab keine signifikanten Unterschiede zwischen den beiden Prüfgruppen (p > 0,05).

**Tabelle 1: Alters- und Geschlechtsverteilung beider Prüfgruppen.**

| | N | MW (Alter) | SD | Range (min-max) | p-Wert |
|---|---|---|---|---|---|
| NADH | 13 | 40,31 | 10,491 | 27-61 | 0,462 |
| Placebo | 14 | 43,71 | 12,964 | 24-61 | |
| NADH W | 9 | 39,89 | 9,239 | 27-57 | 0,811 |
| NADH M | 4 | 41,25 | 14,523 | 27-61 | |
| Placebo W | 9 | 41,33 | 15,215 | 24-61 | 0,384 |
| Placebo M | 5 | 48 | 6,892 | 38-55 | |

| | | | | | |
|---|---|---|---|---|---|
| Legende: M = männlich, W = weiblich, MW = Mittelwert, SD = Standardabweichung, min = Minimum, max = Maximum | | | | | |

Das primäre Interesse der Studie galt der Untersuchung des Einflusses einer einmonatigen NADH-Supplementierung kombiniert mit einem Fitnesstraining auf den allgemeinen Gesundheitszustand und die kognitiven Fähigkeiten. Als sekundäres Ergebnis wurde zudem die Verträglichkeit des Präparates getestet.

### SkillCourt^{®} Shape Jump

Aufgrund von Erkrankungen des Bewegungsapparates (Schmerzen im Kniebereich/Achillesferse) oder weil zu wenig Trainingseinheiten absolviert wurden mussten vier der 27 Teilnehmer/-innen aus der Wertung der motorischen Tests ausgeschlossen werden (*Shape Jump*)*.* 11 Probanden/-innen (acht Frauen, drei Männer) der Verum-Gruppe wurden folglich mit 12 Teilnehmern/-innen (acht Frauen, vier Männer) der Placebo-Gruppe verglichen. Das durchschnittliche Alter der NADH-Gruppe war 39,91 ± 11,44 Jahre (MW ± SD) und das Alter der Placebo-Gruppe betrug im Schnitt 39,5 ± 9,8 Jahre (MW ± SD).

Eine deutliche kognitive Verbesserung konnte bei dem Testverfahren *"Shape Jump"* beobachtet werden. In der NADH-Gruppe erreichten die Teilnehmer/-innen zum Startzeitpunkt zunächst im Schnitt 6.475 ± 453,18 Punkte und verbesserten ihre Leistung nach vier Wochen auf 12.347,73 ± 1.289,81 Punkte, was einer Leistungssteigerung um 90,70% entspricht. In der Placebo-Gruppe konnten sich die Teilnehmer/-innen innerhalb der vier Wochen von anfänglich 6.072,92 ± 528,17 Punkte um 76,74% auf 10.733,33 ± 1.075,43 Punkte verbessern. Die Ergebnisse sind in Fig. 1 dargestellt.

### D2-R

Die Konzentrationsleistung (KL) setzt sich aus den Kennwerten Fehlerprozent (F%) und der Anzahl der bearbeiteten Zielobjekte (BZO) zusammen (KL = BZO-Auslassungs- und Verwechslungsfehler). F% entspricht der relativen Fehlerhäufigkeit (F% = 100 x (Fehler/BZO)). Verglichen wurden die alterskorrigierten Standardwerte (SW) von F%, BZO und KL, die anhand einer Normtabelle ermittelt wurden. Fig. 2 veranschaulicht die Start- und Endwerte der Verum- und der Vergleichs-Gruppe.

Die Fehlerrate F% verbesserte sich in der Chlorophyll-NADH-Gruppe um 6,43% von 89,62 ± 4,46 (Startwert) auf 95,38 ± 4,61% (Endwert). In der Vergleichs-Gruppe betrug die Verbesserung 3,17%. Der Startwert war 84,5 ± 3,93 und der Endwert ergab 87,18 ± 3,60. Der Startwert der BZO in der Chlorophyll-NADH-Gruppe war 92,54 ± 3,20 und stieg innerhalb des Prüfzeitraumes auf einen Endwert von 98,92 ± 3,51 an. In der Vergleichs-Gruppe war eine Steigerung der SW von 87 ± 3,09 auf 93,29 ± 3,38 zu beobachten. Die SW der KL erhöhten sich in der Chlorophyll-NADH-Gruppe von anfangs 89,38 ± 3,82 auf 96,31 ± 4,11 und in der Vergleichs-Gruppe von 83,14 ± 3,60 auf 89,14 ± 3,71.

### SF-36

Der Gesamtgesundheitszustand verbesserte sich innerhalb der vier Wochen in beiden Prüfgruppen: In der NADH-Gruppe verbesserte sich der durchschnittliche Mittelwert ± Standardfehler (MW ± SE) von einem Anfangswert von 125,3 ± 2,51 auf 132,45 ±2,05 um 5,7%. In der Kontrollgruppe war eine Steigerung von 4,60 % von einem Startwert von 126,35 ± 2,76 auf die mittlere finale Punktzahl von 132,16 ± 2,10 zu beobachten. Eine hochsignifikante Verbesserung (p = 0,004) resultierte aus der Sparte "Allgemeine Gesundheitswahrnehmung": Mit einem Cohen's d-Wert von 1,210 und einer Effektstärke von 0,518 (der Effekt hat eine mittlere Stärke) war die allgemeine Gesundheitswahrnehmung in der Chlorophyll-NADH-Gruppe signifikant gesteigert gegenüber der Vergleichs-Gruppe. In der Verum-Gruppe belief sich der Anfangswert auf 19,37 ± 1,04 Punkte und verbesserte sich um 11,84% auf 21,66 ± 0,88, wohingegen sich die Punktzahl in der Kontrollgruppe lediglich um 2,17% von einem Startwert von 21,1 ± 0,75 auf 21,56 ± 0,68 verbesserte. In fünf weiteren Domänen schnitt die Chlorophyll-NADH-Gruppe im Vergleich zu der Vergleichs-Gruppe ebenfalls besser ab: dabei handelt es sich um die Sparten "Körperliche Funktionsfähigkeit", "Körperliche Rollenfunktion", "Vitalität", "Allgemeiner Gesundheitszustand" und "Veränderung Gesundheitszustand".

### Verträglichkeit des Prüfpräparates

Alle Probanden/-innen bewerteten die Prüfsubstanz als gut verträglich. Nach Angaben der Studienteilnehmer/-innen traten durch die Einnahme des Prüfpräparates keinerlei unerwünschte Wirkungen auf.

### Schlussfolgerung

Mit dieser Studie wurde der Einfluss der NADH-Supplementierung mit Sportintervention (also der erfindungsgemäßen Kombination) auf die kognitive Leistungsfähigkeit und den allgemeinen Gesundheitszustand untersucht. Die Vorteile der erfindungsgemäßen Kombination zeigte sich vor allem in einer Verbesserung des Gesamtgesundheitszustands (vor allem in der allgemeinen Gesundheitswahrnehmung) und in der Aufmerksamkeit sowie der Reaktionsgeschwindigkeit. Die erfindungsgemäße Kombination führte zu einer Reduzierung der Fehlerhäufigkeit in diversen kognitiven Bereichen.

## Patentansprüche

1. Verwendung eines NADH-Präparats in Kombination mit einer Sportintervention zur Erhöhung der kognitiven Leistungsfähigkeit oder zur Verbesserung des allgemeinen Gesundheitszustands.

2. Verwendung nach Anspruch 1, wobei die durch Einnahme des NADH-Präparats zugeführte NADH-Tagesdosis 1 mg - 100 mg beträgt, bevorzugt 2 mg - 75 mg, mehr bevorzugt 5 mg - 50 mg, noch mehr bevorzugt 6 mg - 25 mg, insbesondere 7 mg - 15 mg oder gar 8 mg - 12 mg.

3. Verwendung nach Anspruch 1 oder 2, wobei die Sportintervention Herz-Kreislauftraining und/oder Krafttraining umfasst.

4. Verwendung nach einem der Ansprüche 1 bis 3, wobei es sich um eine Verwendung an einer erwachsenen Person handelt.

5. Verwendung nach Anspruch 4, wobei die Person gesund und untrainiert ist.

6. Verwendung nach Anspruch 4 oder 5, wobei die Person über 30 Jahre, bevorzugt über 40 Jahre, alt ist.

7. Verwendung nach einem der Ansprüche 4 bis 6, wobei der Person keine weiteren Präparate zur Nahrungsergänzung zugeführt werden.

8. Verwendung nach einem der vorhergehenden Ansprüche, wobei das NADH-Präparat zumindest ein weiteres Antioxidans zur Stabilisierung des NADH-Präparats umfasst.

9. Verwendung nach Anspruch 8, wobei das weitere Antioxidans Chlorophyll oder Ferredoxin ist.

10. Verwendung nach einem der vorhergehenden Ansprüche, wobei das NADH-Präparat Bienenwachs aufweist.

11. Verwendung nach einem der vorhergehenden Ansprüche, wobei das NADH-Präparat pflanzliches Fett aufweist.

12. Verwendung nach einem der vorhergehenden Ansprüche, wobei das NADH-Präparat in fester Form, insbesondere als Kapsel oder Tablette, vorliegt.

13. Verwendung nach einem der vorhergehenden Ansprüche, wobei das NADH-Präparat eine mikroverkapselte Formulierung aufweist.

14. Verwendung nach einem der vorhergehenden Ansprüche, wobei das NADH-Präparat magensaftresistent formuliert ist.

15. Verwendung nach einem der vorhergehenden Ansprüche, wobei die Verwendung über zumindest 5 Tage, bevorzugt zumindest 10 Tage, noch mehr bevorzugt zumindest über 2 Wochen, insbesondere zumindest über 4 Wochen andauert.
